# EUROPÄISCHE PATENTANMELDUNG

(11) **EP 1 764 369 A1**
(43) Veröffentlichungstag der Anmeldung: **21.03.2007**
(21) Anmeldenummer: 05020208.4
(22) Anmeldetag: 16.09.2005
(51) Int. Cl.: C07K 14/02

(54) **Vakzine enthaltend trunkiertes HBV core protein plus Saponin-basierendes Adjuvants**

(71) Anmelder: Rhein Biotech Gesellschaft für neue biotechnologische Prozesse und Produkte mbH, 40595 Düsseldorf (DE)
(72) Erfinder: Melber, Karl, 40591 Düsseldorf (DE); Buchmann, Pascale, 51061 Köln (DE); Janowicz, Zbigniew, 40699 Erkrath (DE)
(74) Vertreter: Hakvoort, Ansgar

(57) **Zusammenfassung**

Die vorliegende Erfindung betrifft eine Zusammensetzung, beinhaltend:
i) ein HBcAg₁₋ₓ, wobei x eine ganze Zahl aus dem Bereich von 100 bis 160 ist, ein Fragment aus diesem Antigen, eine Variante dieses Antigens oder des Fragmentes dieses Antigens oder mindestens zwei davon
ü) ein Adjuvans beinhaltend ein Saponin oder ein Saponinderivat oder eine Mischung aus mindestens zwei davon,
sowie gegebenenfalls
iii) ein HBsAg, ein Fragment aus diesem Antigen, eine Variante dieses Antigens oder des Fragmentes dieses Antigens oder mindestens zwei davon.

Die Erfindung betrifft auch ein Verfahren zur Herstellung einer Zusammensetzung, die durch dieses Verfahren erhältliche Zusammensetzung, eine Arzneimittelformulierung, die Verwendung der Zusammensetzung oder der Arzneimittelformulierung zur Behandlung und/oder zur Prävention von HBV-Infektionen und HBV-vermittelten Erkrankungen, die Verwendung der Zusammensetzung oder der Arzneimittelformulierung zur Herstellung eines Armeimittels zur Behandlung und/oder zur Prävention von HBV-Infektionen und HHV-veimittelten Erkrankungen sowie ein Verfahren zur Behandlung und/oder zur Prävention von HBV-Infektionen und HBV-vermittelten Erkrankungen.

## Beschreibung

Die vorliegende Erfindung betrifft eine Zusammensetzung, ein Verfahren zur Herstellung einer Zusammensetzung, die durch dieses Verfahren erhältliche Zusammensetzung, eine Arzneimittelformulierung, die Verwendung der Zusammensetzung oder der Arzaeimittelformulierung zur Therapie und/oder zur Prophylaxe von Hepatitis B Virus (HBV)-Infektivnen und HBV-vermittelten Erkrankungen, die Verwendung der Zusammensetzung oder der Arzneimittelibrmulierung zur Herstellung eines Arzneimittels zur Therapie und/oder zur Prophylaxe von HBV-Infektionen und HBV-vermittelten Erkrankungen sowie ein Verfahren zur Therapie und/oder zur Prophylaxe von HBV-Infektionen und HBV-vermittelten Erkrankungen.

Nach Schätzung der Weltgesundheitsorganisation WHO sind oder waren mehr als 2 Milliarden der heute lebenden Menschen mit dem Hepatitis B Virus (HBV) infiziert. Damit gehört HBV zu den bedeutendsten humanpathogenen Erregern mit enormen Auswirkungen auf die menschliche Gesundheit.

Infektionen mit HBV erfolgen über das Blut, besonders von der infizierten Mutter auf das Neugeborene während oder kurz nach der Geburt, durch kontaminierte Blutprodukte und beim Geschlechtsverkehr. HBV stellt ein hepatotropes Virus dar, das sowohl akute wie chronische Infektionen verursachen kann. Während eine Infektion bei der Geburt bzw. in den ersten Lebensmonaten in 90 % der Fälle zu einer chronischen Infektion führt, ist dies bei Infektionen im Erwachsenenalter nur in ca. 10 % der Fälle zu beobachten. In ca. 2 % der infizierten Erwachsenen kann es zu einer fulminanten Hepatitis (akutes Leberversagen) kommen, die mit einer hohen Sterblichkeitsrate verbunden ist

Eine chronische Infektion ist zu Beginn ohne klinische Symptome, jedoch kann sich nach einer Latenzperiode von 10 bis 30 Jahren durch das persistierende HBV und die pathologische Wechselwirkung des Immunsystems mit den infizierten Leberzellen eine akute Lebererkrankung (aktive, chronische Hepatitis B) entwickeln. Der Anteil von chronischen HBV-Trägern, die eine chronische Hepatitis B entwickeln, liegt bei etwa 25 %. Chronische Hepatitis B kann letztendlich zur Leberzirrhose und/oder primärem Leberzellkarzinom führen. Beide Erkrankungen sind mit einer hohen Sterberate verbunden. Es wird geschätzt, dass es weltweit gegenwärtig ca. 350 Millionen chronisch infizierte HBV-Träger gibt. Jedes Jahr sterben ungefähr eine Million Menschen an den Folgen einer chronsichen HBV-Infektion.

Glücklicherweise kann HBV-Infektionen durch prophylaktische Hepatitis B Impfstoffe vorgebeugt werden. Solche Impfstoffe auf der Grundlage von gereinigtem Hepatitis B Obetfilächenantigen (HBsAg) aus dem Plasma chronischer Träger waren erstmals Anfang der Achtziger Jahre verfügbar. Später wurden diese durch rekombinant hergestelltes HBsAg ersetzt. Da jedoch gegenwärtig nicht in allen Ländern die Impfung routinemäßig eingeführt ist, wird HBV auch weiterhin eine Bedrohung der menschlichen Gesundheit darstellen. In diesem Zusammenhang ist es bedeutsam, dass chronische HBV-Träger als infektiös gelten und das Virus übertragen können.

Bei der chronischen Hepatitis B handelt es sich um eine sehr dynamische Erkrankung mit einer Vielzahl von Erscheinungsformen. Im Frühstadium der chronisch verlaufenden Infektion spricht man von der immuntoleranten Phase, die durch die Anwesenheit von viralen Markern im Serum (HBV-DNA, HBsAg, HBeAg) sowie das Fehlen von Leberbeschwerden gekennzeichnet ist. Etwa 25 % der HBV-Träger gehen in eine immunoaktiven Phase über, im Verlaufe derer die ersten Lebersymptome auftreten und eine Therapie erforderlich wird. Während dieser immunoaktiven Phase nehmen HBV-DNA im Serum in der Regel ab und Serum-Transaminasen erhöhen sich schubweise. Während HBsAg weiter im Serum vorliegt, gibt es einen kleinen Prozentsatz (ca. 1 %) von Patienten, die spontan eine Serumkonversion von HBeAg positiv zu HBeAg negativ durchlaufen. Diese Serumkonversion ist Anzeichen für das Eintreten in das inaktive Trägerstadium, das durch eine nur geringe Virusaktivität charakterisiert ist. In wenigen Fällen wird zusätzlich eine Serokonversion von HBsAg positiv zu anti-HBsAg positiv beobachtet, was als Zeichen für eine ausgeheilte Infektion gilt. Die aktive chronische Hepatitis B ist durch Nekroinflammation und Fibrose des Lebergewebes, die im Verlauf der Zeit zur Zirrhose und dekompensierter Leberkrankheit fuhren, gekennzeichnet. Als weitere Komplikation entsteht aus der Zirrhose heraus ein primäres Leberzellkaizinom, wobei manche chronischen HBV-Träger Leberkrebs auch ohne Anzeichen von Zirrhose entwickeln können.

Die Behandlungsmöglichkeiten für aktive chronische Hepatitis B sind sehr beschränkt. Gegenwärtig kann eine Behandlung mit Interferon-α (beispielsweise Intron A^{®} oder Roferon A^{®}) oder antiviralen Substanzen (Lamivudine, Adefovir, Entecavir,) erfolgen. Interferon-α wird bevorzugt bei HBeAg positiven Patienten eingesetzt, wobei ungefähr 30 % mit einer HBeAg-Serumkonversion reagieren, die mit einem inaktiven Trägerstatus korreliert. In der Folge zeigen wenige Patienten (ca. 3 %) auch eine HBsAg-Serokonversion als Anzeichen einer vollständigen Heilung. Die Behandlung mit Interferon-α ist jedoch mit schwerwiegenden Nebenwirkungen verbunden, die oftmals einen vorzeitigen Abbruch der Therapie notwendig machen. In HBeAg negativen Patienten zeigt Interferon-α geringe Ansprechraten verglichen mit HBeAg positiven Patienten. Als Alternative können hier antivirale Substanzen, die zur Klasse der Nukleosid/Nukleotid-Reverse-Transkriptase-Inhibitoren gehören, eingesetzt werden. Diese antiviralen Substanzen unterdrücken die Replikation des Virus und verhindern als Folge ein Fortschreiten der Lebererkrankung. Die Scrumkonversionsraten für HBeAg positive Patienten sind verglichen mit Interferon-α niedriger und liegen nicht höher als die spontan auftretende Remission. Das Ziel der antiviralen Therapie ist daher, die Krankheit durch Verringerung der viralen Aktivität für eine unbegrenzte Zeitspanne aufzuhalten. Der Langzeitgebrauch von antiviralen Substanzen wird jedoch durch das Auftreten von resistenten Virusmutanten gefährdet. Im Falle der am häufigsten eingesetzten Substanz, Lamivudine, wird inzwischen eine Resistenzrate von bis zu 68 % innerhalb von 4 Jahren beobachtet. Das jüngst eingeführte Adefovir scheint Resistenzen mit geringerer Häufigkeit hervorzurufen. Das grundsätzliche Problem der Virusresistenz stellt jedoch eine inhärente Bedrohung für die Langzeitbehandlung dar, selbst wenn die antiviralen Substanzen einmal in Kombination eingesetzt werden sollten. Deshalb besteht ein dringender Bedarf nach zusätzlichen therapeutischen Mitteln zur Behandlung der chronischen Hepatitis B. Insbesondere wünschenswert sind dabei immunomodulatorische Agentien mit geringen Nebenwirkungen und der Perspektive für eine zeitlich begrenzte Therapie für den Patienten.

Eine solche Alternative ist die spezifische Immuntherapie in Form einer therapeutischen Vakzine. Das Konzept einer therapeutischen vakzine ist es, das Immunsystem eines chronischen Hepatitis B Patienten spezifisch so zu stimulieren, dass eine adäquate Immunantwort gegen das Virus initiiert wird, die letztendlich zur Kontrolle über das Virus führt und dem Fortschreiten der Lebererkrankung Einhalt gebietet. Eine der offensichtlichen Wirkungen, die eine therapeutische Vakzine zeigen muss, ist das Durchbrechen der vorliegenden Immuntoleranz in der Gegenwart von großen Mengen an viralen Antigenen. Dabei soll die Therapie insbesondere virus-spezifische T-Zellantworten hervorrufen, insbesondere zytotoxische T-Lymphozyten (CTL) gegen diverse Antigene des HBV. Verglichen mit der Situation bei der akuten, ausheilenden Infektion sind gerade diese Immunantworten in Patienten mit chronischer Hepatitis B gar nicht oder nur sehr schwach nachweisbar. Starke virus-spezifische CTL-Antworten werden allgemein als maßgeblich für die Kontrolle viraler Infektionen angesehen. Die Entwicklung eines therapeutischen Impfstoffes hat daher die Induktion von starken antiviralen CTL-Antworten zum primären Ziel.

Rekombinante Subunit-Impfstoffe sind in der Regel nicht in der Lage CTL-Antworten hervorzurufen. In Tiermodellen (Maus) lassen sich signifikante CTL-Antworten mit DNA-Vakzinen, insbesondere DNA-Vakzinen basierend auf einem Hepatitis B Core Antigen (HBcAg)-kodierenden Plasmidvektor, erzielen, jedoch zeigen derartige Vakzine insgesamt beim Menschen bislang nur sehr schwache Immunantworten und stimulieren keine potente CTL-Antwort. Wegen dieser Schwierigkeiten werden gegenwärtig sogenannte "Prime-Boost-Vakzine" entwickelt, wobei abwechselnd mit einer DNA-Vakzine (z. B. kodierend für HBsAg) und einem rekombinanten Virusvektor (z. B. modifizierter Vaccinia Ankara Stamm), der z. B. das HBsAg exprimiert, immunisiert wird(siehe McConkey SJ, Schneider J, Mendy M, Cavenaugh JS, Bertoletti A, Whittle H, Hill AVS: "Safety and Immunogenicity of a novel "prime boost" therapeutic immunisation strategy against hepatitis B virus", Meeting abstract: "The molecular biology of hepatitis B viruses", Bergamo, Italien, September 7-10,2003).

WO-A-01/98333 beschreibt die Verwendung eines HBc-Antigens, bei dem eine oder mehrere der vier Arginin-Wiederholungseinheiten am C-Terminus entfernt, jedoch der C-terminale Cysteinrest beibehalten wurde, unter anderem zur therapeutischen Behandlung von HBV. Die entfernten Arginin-Wiederholungseinheiten können durch Sequenzen ersetzt sein, welche Epitope aus bakteriellen oder viralen Pathogenen, Parasiten, Allergenen oder mit Krebs assoziierten Antigenen repräsentieren, die von T-Helferaellen, B-Zellen oder zytotoxischen T-Lymphozyten erkannt werden.

WO-A-2005/023297 beschreibt eine Zusammensetzung zur Behandlung von HBV-Infektionen und HBV-vermittelten Erkrankungen, die wenigstens zwei Hepatitis B Oberflächenantigene (HBsAg's), Fragmente davon und/oder diese kodierende Nukleinsäuren enthalten, wobei sich die HBsAg's im HBV-Genotyp in der S-Region und/oder der Prä-S 1-Region unterscheiden und wobei die Zusammensetzung kein HBcAg oder dieses kodierende Nukleinsäuren enthält.

WO-A-2005/056051 offenbart unter anderem eine rekombinante Polypeptidzusammensetzung zur Behandlung oder Prophylaxe einer Hepatitis B-Infektion, wobei die Zusammensetzung eine Vielzahl von Komponenten, vorzugsweise mindestens drei oder alle vier ausgewählt aus a) einem Core-Polypeptid-Element, welches eine Sequenz der immunogenen Region des HBV-Core-Proteins beinhaltet, b) einem Oberflächenantigen-Polypeptid-Element, welches eine Sequenz der immunogenen Region des HBV-Oberflächenantigen-Proteins (S, M oder L) beinhaltet, c) einem X-Polypeptid-Element, welches eine Sequenz der immunogenen Region des HBV-X-Proteins beinhaltet und d) eines Polymerase-Polypeptid-Elements, welches eine Sequenz der immunogenen Region des HBV-Polymerase-Proteins beinhaltet, wobei mindestens eines der Elemente mindestens 100 Aminosäuren des jeweiligen HBV-Proteins umfasst.

EP-A-1 136 077 offenbart eine Impfstoffzusammensetzung für die nasale Anwendung, umfassend ein HBsAg und gegebenenfalls ein HBcAg, welche unter anderem auch als therapeutische Vakzine eingesetzt werden kann.

Der vorliegenden Erfindung lag die Aufgabe zugrunde, die sich aus dem Stand der Technik ergebenden Nachteile von Impfstoffen zur Behandlung oder Prophylaxe einer Hepatitis B-Infektion beim Menschen zu überwinden.

Insbesondere lag der vorliegenden Erfindung die Aufgabe zugrunde, eine für die Behandlung eines Menschen geeignete Zusammensetzung anzugeben, welche im Vergleich zu den aus dem Stand der Technik bekannten Zusammensetzungen zu stärkeren Immunantworten, insbesondere starken CTL-Antworten gegen das HBcAg, in Patienten mit einer chronischen HBV-Infektion führt. Dabei sollte die Zusammensetzung bei möglichst vielen Patienten mit einer chronischen HBV-Infektion, unabhängig von deren HLA-Typ, zu einem Durchbrechen der immunologischen Toleranz führen.

Dabei soll die erfindungsgemäße Zusammensetzung unabhängig vom HBV-Genotyp des chronsichen Patienten ein Durchbrechen der immunologischen Toleranz ermöglichen. und damit zur Ausbildung einer das Virus kontrollierenden Immunantwort führen.

Der vorliegenden Erfindung lag weiterhin die Aufgabe zugrunde, ein Verfahren anzugeben, mit dem mit möglichst geringem präparativen Aufwand eine Zusammensetzung mit den vorstehend beschriebenen Vorteilen erhalten werden kann.

Einen Beitrag zur Lösung der eingangs genannten Aufgaben leistet eine Zusammensetzung, beinhaltend:
i) ein HBcAg₁₋ₓ (HBcAg = HBV core-Antigen), wobei x eine ganze Zahl aus dem Bereich von 100 bis 160, vorzugsweise dem Bereich von 120 bis 150, besonders bevorzugt dem Bereich von 140 bis 149, darüber hinaus bevorzugt dem Bereich von 144 bis 146 und am meisten bevorzugt gleich 145 ist, ein Fragment aus diesem Antigen, eine Variante dieses Antigens oder des Fragmentes dieses Antigens oder eine Mischung aus mindestens zwei davon,
ii) ein Adjuvans beinhaltend ein Saponin oder ein Saponinderivat oder eine Mischung aus mindestens zwei davon,
   sowie gegebenenfalls
iii) ein HBsAg, ein Fragment aus diesem Antigen, eine Variante dieses Antigens oder des Fragmentes dieses Antigens oder eine Mischung aus mindestens zwei davon.

Völlig überraschend, dafür aber nicht minder vorteilhaft, wurde festgestellt, dass sich mit der vorstehend beschriebenen, für einen Einsatz beim Menschen grundsätzlich geeigneten Zusammensetzung starke, core-spezifische CTL-Antworten im Tiermodell erhalten lassen, welche in etwa vergleichbar waren mit denjenigen CTL-Antworten, die bei der Verwendung eines HBcAg-kodierenden Plasmidvektors (DNA-Immunisierung) im gleichen Tiermodell erhalten wurden.

Der in den folgenden Ausführungen verwendete Begriff "Polypeptid" betrifft ein Polymer aus Aminosäuren und ist nicht auf eine bestimmte Mindestzahl an Aminosäuren beschränkt. Er umfasst daher ebenso Peptide, Oligopeptide, Dimere, Trimere, Oligomere, Partikel und dergleichen. Weiterhin umfasst der Begriff "Polypeptide" nicht nur die reinen Polymere aus Aminosäuren, welche unmittelbar nach der Translation in den Ribosomen erhalten werden, sondern auch diejenigen Polypeptide, die posttranslational durch Glykosilierung, Acetylierung, Phosphorylierung und dergleichen erhalten werden.

Unter einem "HBcAg₁₋ₓ" wird erfindungsgemäß ein Polypeptid verstanden, welches die Aminosäuren 1 bis x des natürlichen HBcAg's umfasst, wobei unter der Bezeichnung "HBcAg₁₋ₓ" alle denkbaren Polypeptide mit den Aminosäuren 1 bis x (wobei die erste Aminosäure (= Aminosäure 1) diejenige am N-tenninalen Ende des Polypeptides ist) des HBcAg's der zur Zeit bekannten 8 Standardgenotypen A, B, C, D, E, F, G und H zu verstehen sind. Diese 8 Standardgenotypen unterscheiden sich in ca. 8 % ihrer Nukleotidsequenz voneinander (siehe Bartholomeusz, Rev. Med. Virol. 13 (2003), 1-14), wobei die Variation vor allem das HBsAg-Gen betreffen. Vorzugsweise weist der HBV Genotyp A die Referenz-Nukleinsäuresequenz Genbank X02763 bzw. der HBV Genotyp A_{afr} die Referenz-Nukleinsäuresequenz gemäß Genbank AF297621 auf. Für den HBV Genotyp Bₐ ist die Referenz-Nukleinsäuresequenz Genbank D00330, für den Genotyp Bⱼ die Referenz-Nukleinsäuresequenz AB073S58. Für den HBV Genotyp C ist die Referenz-Nukleinsäuresequenz Genbank AY206389, für den Genotyp Cₐᵤₛ die Referenz-Nukleinsäuresequenz gemäß Genbank AB048704. Für den Genotyp D ist die Referenz-Nukleinsäuresequenz Genbank X02496, für den Genotyp E Genbank X75657, für den Genotyp F Genbank X69798, für den Genotyp G Genbank AF160501 und für den Genotyp H Genbank AY090454.

Unter der Bezeichnung "Fragment" eines HBcAg₁₋ₓ's wird vorzugsweise ein Polypeptid verstanden, bei dem ein Abschnitt von mindestens 25, vorzugsweise mindestens 50 und besonders bevorzugt mindestens 100 Aminosäuren identisch ist mit einem entsprechenden Abschnitt des HBcAg₁₋ₓ's.

Unter der Bezeichnung "Variante" des HBcAg₁₋ₓ's oder des Fragmentes des HBcAg₁₋ₓ's wird vorzugsweise ein Polypeptid verstanden, bei dem höchstens 10, vorzugsweise höchstens 5 Aminosäuren, besonders bevorzugt höchstens 2 Aminosäuren und am meisten bevorzugt höchstens 1 Aminosäure des HBcAg₁₋ₓ's oder des Fragmentes des HBcAg₁₋ₓ's deletiert, insertiert, substituiert oder aber an das C- und/oder N-terminale Ende angefügt, vorzugsweise substituiert sind bzw. ist. Die Bezeichnung "Variante" umfasst auch Fusionspolypeptide , insbesondere Fusionen mit HBsAg, welche HBcAg₁₋ₓ enthalten.

Unter dem Begriff "Adjuvans" wird erfindungsgemäß eine Verbindung verstanden, welche die Induktion einer Immunantwort, vorzugsweise die Induktion einer CTL-Antwort auf das Antigen ermöglicht.

Die in der erfindungsgemäßen Zusammensetzung enthaltenen, gegenüber den natürlichen HBcAg's trunkierten HBcAg₁₋ₓ's, denen zumindest ein Teil der C-terminalen Nukleinsäurebindungsregion, vorzugsweise die gesamte C-terminale Nukleinsäurebindungsregion fehlt, können durch dem Fachmann bekannte Rekombinationsverfahren erhalten werden.

Eine Möglichkeit besteht darin, das core- bzw. pre-core-Leseraster mittels Polymerasekettenreaktion (PCR) aus einem Virusisolat zu amplifizieren. Denkbar ist auch, die entsprechende DNA-Sequenz auf synthetischem Weg herzustellen und anschließend mittels PCR zu amplifizieren. Das erhaltene PCR-Fragment wird dann in einen Plasmidvektor kloniert. Mittels weiterer PCR-Klonierungen kann dann die entsprechend verkürzte Form in einen Expressionsvektor, beispielsweise in einen Expressionsvektor für E. coli, insertiert werden. Dabei erfolgt die Insertion vorzugsweise dergestalt, dass das für das HBcAg₁₋ₓ bzw. für das Fragment oder die Variante kodierende Gen unter der Kontrolle eines aktiven Promoters steht. Der Expressionsvektor kann gleichzeitig Induktionselemente zur Erhöhung der Expressionsrate enthalten. Nach der Transformation dieses Expressionsvektors in E. coli werden Einzelklone erhalten, welche das HBcAg₁₋ₓ bzw. das Fragment oder die Variante exprimieren. Um die als Antigene dienenden Polypeptide zu erhalten, wird ein Einzelklon in Kulturmedium angeimpft und über Nacht bei 37°C im Schüttelkolben kultiviert. Die E. coli-Kultur wird dann zunächst durch Zentrifugation abgeemtet. Das Bakterienpellet wird in Pufferlösung resuspendiert und anschließend entweder durch Ultraschallbehandlung oder durch Hochdruckhomogenisation aufgeschlossen. Das im Homogenisat befindliche HBcAg bzw. HBcAg-Variante wird durch Fällung mit Ammoniumsulfat konzentriert und anschließend durch Gelfiltration von bakteriellen Wirtskomponenten gereinigt. Partikelbildende HBcAg-Varianten können weiter über eine Geschwindigkeits-Zonenzentrifugation aufgereinigt werden. Partikeldefiziente HBcAg₁₋ₓ's werden idealerweise mit einem N-terminalen His-Tag-Affinitätsmarker konstruiert und mittels einer Ni-NTA-Affinitätschromatographie und anschließender Gelfiltration aufgereinigt. Die so hergestellten HBcAg₁₋ₓ's werden steril filtriert und als solche für die Formulierung der erfindungsgemäßen Zusammensetzung eingesetzt.

Grundsätzlich können die in der erfindungsgemäßen Zusammensetzung enthaltenen HBcAg₁₋ₓ's bzw. deren Fragmente oder Varianten auch auf synthetischem Weg erhalten werden.

Weitere Hinweise zu den Techniken der rekombinanten oder synthetischen Herstellung von Polypeptiden können
- Sambrook, "Molecular Cloning: A Laboratory Manual", Zweite Auflage (1989);
- *"*DNA Cloning", Volumes I Und II (D. N. Glover, Herausgeber., 1985);
- *"*Oligonucleotide Synthesis" (M. J. Gait, Herausgeber, 1986);
- *"*Nucleic Acid Hybridization" (B. D. Hames & S. J. Higgins, Herausgeber, 1984);
- *"*Transcription and Translation" (B. D. Hames & S. J. Higgins, Herausgeber, 1984);
- *"*Animal Cell Culture" (R. I. Freshney, Herausgeber, 1986);
- *"*Immobilized Cells and Enzymes" (IRL Press, 1986);
- B. Perbal, "A Practical Guide to Molecular Cloning" (1984);
- *"*The Methods in Enzymology series" (Academic Press, Inc.), insbesondere Volumes 154 und 155;
- *"*Gene Transfer Vectors for Mammalian Cells" (J. H. Miller und M. P. Calos, Herausgeber, 1987, Cold Spring Harbor Laboratory);
- Mayer und Walker, Herausgeber, (1987), "Immunochemical Methods in Cell and Molecular Biology" (Academic Press, London);
- Scopes, (1987), "Protein Purification: Principles and Practice", Zweite Auflage (Springer-Verlag, N.Y.) und
- *"*Handbook of Experimental Immunology", Volumes I-IV (D. M. Weir und C. C. Blackwell, Herausgeber, 1986)
entnommen werden.

Grundsätzlich können die in der erfindungsgemäßen Zusammensetzung enthaltenen HBcAg₁₋ₓ's partikelbildend oder nicht partikelbildend sein, wobei partikelbildende HBcAg₁₋ₓ's bevorzugt sind. Partikelbildende HBcAg₁₋ₓ's sind in der Lage, zu Kapsiden mit einem Durchmesser von ca. 27 nm zu assoziieren. Dabei lagern sich 180 bzw. 240 Dimere zu makromolekularen Strukturen zusammen.

Erfindungsgemäß bevorzugte Antigene sind insbesondere das HBcAg₁₋₁₂₄, das HBcAg₁₋₁₂₇, das HBcAg₁₋₁₄₄, das HBcAg₁₋₁₄₅, das HBcAg₁₋₁₄₆, das HBcAg₁₋₁₄₇, das HBcAg₁₋₁₄₈, das HBcAg₁₋₁₄₉, (also Polypeptide, welche die ersten 127, 144, 145, 146, 147, 148 bzw. 149 Aminosäuren des natürlichen FiBcAg's umfassen) und HBeAg₈₁₋₁₃₃ (ein Fragment des HBcAg's, welches die Aminosäuren 81 bis 133 des natürlich vorkommenden HBcAg's umfasst). Ein weiterhin bevorzugtes Antigen ist HBcAg_{1-144+AS}, ein Polypeptid, welches die ersten 144 Aminosäuren des natürlich vorkommenden HBcAg's umfasst und welches als 145. Aminosäure eine von der im natürlich vorkommenden HBcAg als 145. Aminosäure auftretenden Glutaminsäure verschiedene Aminosäure AS aufweist, wobei es sich bei dieser von Glutaminsäure verschiedenen Aminosäure AS vorzugsweise um Isoleucin handelt. In der erfindungsgemäßen Zusammensetzung besonders bevorzugt enthaltene Antigene sind HBcAg₁₋₁₄₅ und HBcAg₁₋₁₄₄₊₁.

Weiterhin ist es erfindungsgemäß bevorzugt, dass das HBcAg₁₋ₓ keine HBcAgfremden Epitope, insbesondere keine HBsAg-Epitope aufweist. Auch ist es gemäß einer besonderen Ausführungsform der erfindungsgemäßen Zusammensetzung bevorzugt, dass das HBcAg₁₋ₓ, sofern x nicht gleich 48, 61 bzw. 107 ist, an der C-terminalen Position x keinen Cystein-Rest aufweist.

Gemäß einer besonderen Ausführungsform der erfindungsgemäßen Zusammensetzung beinhaltet diese mindestens zwei HBcAg₁₋ₓ's oder Fragmente oder Varianten davon, wobei sich die HBcAg₁₋ₓ's im HBV-Genotyp unterscheiden.

Die erfindungsgemäße Zusammensetzung umfasst ein Adjuvans beinhaltend ein Saponin oder ein Saponinderivat oder eine Mischung aus mindestens zwei davon. Vorzugsweise handelt es sich bei diesem Adjuvans um einen Saponinkomplex, einen Saponinderivatkomplex oder eine Mischung aus mindestens zwei davon. Besonders bevorzugt handelt es sich bei dem Saponinkomplex bzw. dem Saponinderivatkomplex um einen liposomalen Saponinkomplex bzw. liposomalen Saponinderivatkomplex.

In diesem Zusammenhang ist es weiterhin bevorzugt, dass der Saponinkomplex, der Saponinderivatkomplex oder die Mischung aus mindestens zwei davon als von Wasser oder wässrigen Salzlösungen verschiedene Komponenten
(α1) 0 bis 95 Gew.-%, besonders bevorzugt 10 bis 50 Gew.-% und am meisten bevorzugt 15 bis 25 Gew.-% eines Phospholipids,
(α2) 0 bis 95 Gew.-%, besonders bevorzugt 10 bis 50 Gew.-% und am meisten bevorzugt 15 bis 25 Gew.-% eines Steroids,
(α3) 5 bis 100 Gew.-%, besonders bevorzugt 15 bis 90 Gew.-% und am meisten bevorzugt 30 bis 80 Gew.-% des Saponins bzw. des Saponinderivates, sowie
(α4) 0 bis 20 Gew.-%, besonders bevorzugt 1 bis 15 Gew.-% und am meisten bevorzugt 5 bis 10 Gew.-% weitere Zusatzstoffe, beispielsweise weitere, von Phospholipiden und Steroid-Lipiden verschiedene Lipide oder von Wasser verschiedene Lösungsmittel
beinhaltet, wobei die Gewichtsmengen jeweils auf das Gesamtgewicht des Saponinkomplexes bzw. des Saponinderivatkomplexes, mit Ausnahme seines Wasseranteils bzw. seines Anteils an wässrigen Salzlösungen, bezogen sind und die Summe der Komponenten (α1) bis (α4) 100 Gew.-% beträgt.

Derartige Saponinkomplexe werden in der Literatur unter anderem auch als "ISCO-Matrix" bezeichnet. Beinhaltet die Zusammensetzung neben den Komponenten (α1) bis (α4) auch Proteine als Antigene, so bezeichnet man den Komplex als "ISCOM^{®}".

Bei den Phospholipiden (α1) handelt es sich vorzugsweise um Phospholipide ausgewählt aus der Gruppe bestehend aus Phosphatidylcholin, Phosphatidylethanolamin und Dipalmitoylphosphatidylcholin.

Bei den Steroiden (α2) handelt es sich vorzugsweise um Steroide pflanzlichen oder tierischen Ursprungs ausgewählt aus der Gruppe bestehend aus Cholesterin, Lanosterin, Lumisterin, Stigmasterin und Sitosterin.

Als Saponine werden erfindungsgemäß vorzugsweise chemische Verbindungen zwischen einem Zucker und einem Steroid, Steroidalkaloid oder Triterpen oder Mischungen aus diesen Verbindungen verstanden. Man spricht auch von Steroidsaponinen, Steroidalkaloidsaponinen und Triterpensaponinen. Solche Saponine sind oberflächenaktive Glykoside und können aus Pflanzen gewonnen werden, wobei die als Adjuvans am stärksten wirksamen Saponine vom südamerikanischen Baum *Quillaja saponaria,* vorzugsweise aus der Rinde dieses Baums gewonnen werden. Bei der Isolierung der Saponine aus *Quillaja saponaria* wird ein Extrakt erhalten, der in teilweise aufgereinigter Form als "Quil A" bezeichnet wird (der teilweise aufgereinigte Saponinextrakt hat einen definierten Triterpensaponin-Gehalt und ist weniger toxisch als der Roh-Saponinextrakt).

Weitere geeignete Saponine sind beschrieben in R. Tschesche und Wulf, "Chemie und Biologie der Saponine in Fortschritte der Chemie Organischer Naturstoffe", veröffentlicht durch H. Herz, H. Grisebach, G. W. Kirby, Vol. 30 (1973), insbesondere stark polare Saponine wie die polaren Triterpensaponine, insbesondere die polaren, saueren Bisdesmoside, etwa der Saponinextrakt aus *Quillajabark Aralosid A,* Chikosetsusaponin IV, Calendula-Glycosid C, ChikoseL-ewaponin V, Achyranthes-Saponin B, Calendula-Glycosid A, Aralosid B, Aralosid C, Putranjia-Saponin III, Bersamasaponisid, Putrajia-Saponin IV, Trichosid A, Trichosid B, Saponasid A, Trichosid C, Gypsosid, Nutanosid, Dianthosid C, Saponasid D, vorzugsweise Aescin aus *Aesculus hippocastanum* (T Patt und W. Winkler:: "Das therapeutisch wirksame Prinzip der Rosskastanie (Aesculus hippocastanum)", Arzneimittelforschung 10(4), 273-275 (1960).

Bei dem Saponin (α3) handelt es sich vorzugsweise um den als "Quil A" bezeichneten, teilweise aufgereinigten Saponinextrakt aus der Rinde von *Quillaja saponaria,* der aus mindestens 22 verschiedenen Saponinfraktionen besteht, um einzelne Unterfraktionen dieser homogenen Quil A-Fraktion, wie beispielsweise QS 7, QS 17, QS 18 oder QS 21, oder um eine Mischung aus mindestens zwei der aus der homogenen Quil A-Fraktion erhaltenen Unterfraktionen, vorzugsweise um eine Mischung aus den Unterfraktionen A und C, wie sie in WO-A-96/11711 beschrieben ist. Diese Mischung besteht vorzugsweise aus 50 bis 90 Gew.-% der Fraktion A und 10 bis 50 Gew.-% der Fraktion C, wobei die Fraktionen A und C gemäß dem Beispiel 1 der WO-A,-96/11711 erhalten werden. Solche Mischungen sind beispielsweise unter der Bezeichnung "ISCOPREP^{®}703 kommerziell erhältlich. Gemäß einer besonderen Ausführungsform kann das Saponin auch zu 100 Gew.-% aus der Fraktion C bestehen. Neben dem Saponin aus Quil A können auch Saponinderivate, wie sie beispielsweise in US 6,262,029 beschrieben sind, im Komplex enthalten sein. Erfindungsgemäß geeignete Saponinkomplexe sind insbesondere die kommerziell erhältlichen Produkte AbISCO®-100, AbISCO®-200, AbISCO^{®}-300 (ISCONOVA, Uppsala, Schweden) oder Stimulon^{®}QS-21 (ANTIGENICS. Woburn, USA).

Die Herstellung der vorstehend beschriebenen Saponinkomplexe (ISCO-Matrix) kann durch jedes dem Fachmann bekannte Verfahren zur Herstellung solcher Komplexe erfolgen. Bevorzugte Verfahren zur Herstellung derartiger Saponinkomplexe sind beispielsweise in EP-A-0 231 039 (liposomale Saponinkomplexe) oder in WO-A-90/03184 (nicht liposomale Saponinkomplexe) beschrieben, deren Offenbarungsgehalt hinsichtlich der Herstellung einer ISCO-Matrix hiermit als Referenz eingeführt wird und einen Teil der Offenbarung der vorliegenden Erfindung bildet.

Sofern in der erfindungsgemäßen Zusammensetzung eine ISCO-Matrix als Adjuvans enthalten ist, ist es erfindungsgemäß weiterhin bevorzugt, dass das Antigen bzw. die Antigene nicht im Inneren der Isco-Matrix lokalisiert ist bzw. sind, wie diese bei den bekannten ISCOM's der Fall ist.

Gemäß einer besonderen Ausführungsform der erfindungsgemäßen Zusammensetzung kann diese neben dem Saponinkomplex, dem Saponinderivatkomplex oder der Mischung aus mindestens zwei dieser Komplexe auch weitere, kein Saponin oder Saponinderivat beinhaltende Adjuvantien als Co-Adjuvantien beinhalten, wobei diese Co-Adjuvantien getrennt von dem Komplex vorliegen oder aber in diesen Komplex eingebunden sein können. Als Co-Adjuvans kann jedes dem Fachmann bekannte Adjuvans eingesetzt werden, welches üblicherweise in Vakzinen eingesetzt wird. Bevorzugte Adjuvantien sind ausgewählt aus der Gruppe umfassend partikuläre Adjuvantien wie beispielsweise Submikro-ÖI-in-Wasser-Emulsion, zu denen etwa MF59 (5% Squalen, 0,5% Tween 80 und 0,5% Span 85), SAF (10 % Squalen, 0,4 % Tween 80, 5 % Pluron-blockiertes Polymer sowie thr-Muramyldipeptid (thr-MDP) gehören, Mineralverbindungen wie etwa Aluminiumhydroxid, Aluminiumsulfat oder Aluminiumphosphat, unvollständiges Freundsches Adjuvans, Lipopolysaccharide, N-Acetylglucosaminyl-N-aeetylmuramyl-L-alanyl-Dipeptid (GMDP) oder Muiramyldipeptid (MDP), GAL-Ceramid, Dimethyldioeta-decylammoniumbromid (DDAB), Liposomen, vorzugsweise unfassend Phospholipid und Cholesterin, Mikropartikel aus biologisch abbaubaren Polymeren wie Poly-Lactid-co-glykolid (PLGA), Oligodesoxyribonukleotide mit oder ohne CpG-Motiv, N,N-Di-(β-Stearoylethyl)-N,N-dimethylammonium-chlohd (EQ1), Glykopeptide, Bestandteile der Zellwand von Mycobakterien, quaternäre Amine wie beispielsweise Cetylpyridiniumchlorid und Benzalkoniumchlorid, zwitterionische Amine wie CHAPS (3-(3-Cholamidopropyl)-dimethyl-ammonio)-1-propansulfonat), Dextransulfat, Granulozyten-Makrophagen-Kolonien stimulierender Faktor (GM-CSF), Tumomekrosefaktor (TNF), , Blockcopolymere wie beispielsweise P 1205 oder Poloxamer 401, Dimyristoylphosphatidylcholin (DMPC), 3β-Hydroxy-5-androsten-17-on (DHEA), Dimyristoylphosphatidyl-glycerol (DMPG), Desoxycholsäure-Natriumsalz, Imiquimod, DTP-GDP, 7-Allyl-8-Oxoguanosin, Montanide ISA 51, Montanide ISA 720, Murametid, Murapalmitin, Dicetylphosphat, Polymethylmethacrylat (PMMA), PEG-Svrbitanfettsäureester wie Polysorbat 20 oder80 (TWEEN 20,80), detoxifizierte Mutanten von bakteriellen ADP-ribosylierenden Toxinen wie Cholera-Toxin oder Pertussis-Toxin oder Mischungen aus mindestens zwei dieser Adjuvantien. Als Co-Adjuvantien besonders bevorzugt sind Lipopolysaccharide, Aluminiumhydroxid, Aluminiumphosphat, Gal-Ceramid, Oligodesoxyribonukleotide mit CpG-Motiv, sowie PEG-Sorbitanfettsäureester.

Eine Übersicht über als Co-Adjuvantien geeignete Verbindungen ist in Cox, J.C. und Coulter, A.R, "Adjuvants-α classification and review of their modes of action" in Vaccine 15;248-256 (Febr. 1997) dargestellt. Die Offenbarung dieser Druckschrift hinsichtlich möglicher Adjuvantien in Impfstoffzusammensetzungen wird hiermit als Referenz eingeführt und bildet einen Teil der Offenbarung der vorliegenden Erfindung.

Gemäß einer weiteren besonderen Ausführungsform der erfindungsgemäßen Zusammensetzung enthält diese neben dem Antigen HBcAg₁₋ₓ und dem Adjuvans noch mindestens ein HBsAg, ein Fragment aus diesem Antigen, eine Variante dieses Antigens oder des Fragmentes dieses Antigens oder eine Mischung aus mindestens zwei davon.

Bei dem HBsAg handelt es sich um ein 226-Aminosäuren Protein (p24/gp27 oder S-Prvtein), das in 20-30 nm Lipoproteinpartikel selbstassembliert, in denen ungefähr 100-150 Untereinheiten durch multiple inter-und intra-molekulare Disulfidbindungen quervernetzt sind. Die in der erfindungsgemäßen Zusammensetzung gegebenenfalls enthaltenen HBsAg's können dabei, ebenso wie die HBcAg₁₋ₓ's, von allen zur Zeit bekannten 8 HBV-Standardgenotypen A, B, C, D, E, F, G und H abgeleitet sein. Der Begriff "HBsAg" umfasst weiterhin neben dem 226-Aminosäuren Protein auch die beiden M- und L-Proteine, die neben dem HBsAg noch das 55-Aminosäuren lange Prä-S2-Peptid (M-Protein) bzw. das 55-Aminosäuren lange Prä-S2-Peptid und das 120-Aminosäuren lange Prä-S1-Peptid (L-Protein) umfassen.

Unter der Bezeichnung "Fragment" eines HBsAg's wird vorzugsweise ein Polypeptid verstanden, bei dem ein Abschnitt von mindestens 50, vorzugsweise mindestens 150 und besonders bevorzugt mindestens 200 Aminosäuren identisch ist mit einem entsprechenden Abschnitt des HBsAg's.

Unter der Bezeichnung "Variante" des HBsAg's oder des Fragmentes des HBsAg's wird vorzugsweise ein Polypeptid verstanden, bei dem höchstens 5, vorzugsweise höchstens 2 und besonders bevorzugt höchstens 1 Aminosäure des HBsAg's oder des Fragmentes des HBsAg's deletiert, insertiert, an den Enden angefügt oder substituiert, vorzugsweise substituiert ist.

Gemäß einer bevorzugten Ausführungsform der erfindungsgemäßen Zusammensetzung, bei der diese mindestens ein HBsAg enthält, umfasst diese nicht nur ein HBsAg, sondern, wie dies in der WO-A-2405/02397 beschrieben ist, wenigstens zwei HBsAg's oder Fragmente oder Varianten davon, wobei sich die HBsAg's im HBV-Genotyp in der S-Region und/oder der Prä-S1-Region unterscheiden.

Die HBsAg's können entweder aus dem Serum von Patienten mit chronischer Hepatitis B, auf synthetischem Wege oder als rekombinante Polypeptide durch dem Fachmann bekannte Verfahren erhalten werden. In diesem Zusammenhang sei auf die vorstehend beschriebenen Methoden im Zusammenhang mit der Gewinnung der HBcAg₁₋ₓ verwiesen.

Die erfindungsgemäße Zusammensetzung kann, insbesondere dann, wenn sie als Arzneimittelformulierung vorliegt, neben den Komponenten i), ii) und gegebenenfalls iii) auch noch weitere Zusatzstoffe iv), wie Arzneistoffträger oder Hilfsstoffe beinhalten.

Bevorzugte ArzneistoHträger sind insbesondere Wasser, gepuffertes Wasser, vorzugsweise isotonische Salzlösungen wie PBS *(Phosphate Buffered Saline),* Glucose, Dextrose, Mannitol, Lactose, Stärke, Magnesiumstearat, Zellulose, Magnesiumcarbonat, 0,3 % Glycerol, Hyaluronsäure, Ethanol, Polyalkylenglykole wie Polypropylenglykol, Triglyceride oder dergleichen. Die Art des zugesetzten Arzneistoffträgers hängt insbesondere davon ab, ob die erfindungsgemäße Zusammensetzung für eine orale, eine intranasale, eine intradermale, eine subkutane, eine intramuskuläre oder eine intravenöse Verabreichung formuliert wird. Als Hilfsstoffe können Tonizitätsregler, Benetzungsmittel, Emulgierhilfen oder Puffersubstanzen in der erfindungsgemäßen Zusammensetzung enthalten sein.

Das relative Mengenverhältnis zwischen der Antigenkomponente i) bzw. den Antigenkomponenten i) und iii) zum Adjuvans ii) liegt vorzugsweise in einem Bereich von 1 : 20 bis 20 : 1, besonders bevorzugt in einem Bereich von 1 : 10 bis 10 : 1, darüber hinaus bevorzugt in einem Bereich von 1 : 5 bis 5 : 1 und am meisten bevorzugt in einem Bereich von 1 : 2 bis 2 : 1. Das für den jeweiligen Anwendungsfall optimale Gewichtsverhältnis zwischen Adjuvans und Antigen kann der Fachmann durch einfache Vorversuche, bei denen beispielsweise die Stärke der CTL-Antwort in Abhängigkeit von diesem Mengenverhältnis bestimmt wird, ermitteln.

Sofern die erfindungsgemäße Zusammensetzung neben dem HBcAg₁₋ₓ auch ein HBsAg umfasst, so liegt das Gewichtsverhältnis zwischen dem HBcAg₁₋ₓ und dem HBsAg vorzugsweise in einem Bereich von 1 : 20 bis 20 : 1, besonders bevorzugt in einem Bereich von 1 : 10 bis 10 : 1, darüber hinaus bevorzugt in einem Bereich von 1 : 5 bis 5 : 1 und am meisten bevorzugt in einem Bereich von 1 : 2 bis 2 : 1. Auch hier kann der Fachmann das optimale Gewichtsverhältnis durch einfache Vorversuche ermitteln.

Die Gesamtkonzentration der Komponenten i), ii) und gegebenenfalls iii) in der erfindungsgemäßen Zusammensetzung (also die Gesamtmenge der Komponenten i), ii) und iii) bezogen auf das Volumen der erfindungsgemäßen Zusammensetzung) liegt, sofern die erfindungsgemäße Zusammensetzung nicht als Lyophylisat vorliegt, vorzugsweise in einem Bereich von 0,1 bis 2.000 µg/ml, besonders bevorzugt in einem Bereich von 0,5 bis 1.500 µg/ml, darüber hinaus bevorzugt in einem Bereich von 1 bis 1.000 µg/ml und am meisten bevorzugt in einem Bereich von 10 bis 500 µg/ml.

Gemäß einer Ausführungsform der erfmdungsgemäßen Zusammensetzung umfasst diese
i) ein HBeAg₁₋₁₄₅ sowie
ii) einen Saponinkomplex als Adjuvans, vorzugsweise einen liposomalen Saponinkomplex.

Gemäß einer anderen Ausführungsform der erfindungsgemäßen Zusammensetzung umfasst diese
i) ein HBcAg₁₋₁₄₄₊₁ sowie
ii) einen Saponinkomplex als Adjuvans, vorzugsweise einen liposomalen Saponinkomplex.

Gemäß einer weiteren Ausführungsform der erfindungsgemäßen Zusammensetzung umfasst diese
i) ein HBcAg₁₋₁₄₅,
ii) einen Saponinkomplex als Adjuvans, vorzugsweise einen liposomalen Saponinkomplex, sowie
iii) ein HBsAg.

Gemäß einer weiteren Ausführungsform der erfindungsgemäßen Zusammensetzung umfasst diese
i) ein HBcAg₁₋₁₄₄₊₁,
ii) einen Saponinkomplex als Adjuvans, vorzugsweise einen liposomalen Saponinkomplex, sowie
iii) ein HBsAg.

Vorzugsweise ist die erfindungsgemäße Zusammensetzung des weiteren dadurch gekennzeichnet, dass sie in C57B16-Mäusen bei einer Immunisierung gemäß dem hierin beschriebenen Immunisierungsverfahren und anschließender, hierin beschriebener Restimulierung mit MGLKFRQL (einem synthetischen Fragment des HBcAg's) eine gemäß der hierin beschriebenen Methode bestimmte HBcAgspezifische CD8⁺-T-Zell-Frequenz in der Milz von mindestens 30, besonders bevorzugt mindestens 50, darüber hinaus bevorzugt mindestens 75, darüber hinaus noch mehr bevorzugt mindestens 100 und am meisten bevorzugt mindestens 300 CD8⁺ IFN_{γ}⁺ T-Zellen pro 10⁵ CD8⁺ T-Zellen aufweist.

Weiter ist die erfindungsgemäße Zusammensetzung vorzugsweise dadurch charakterisiert, dass sie, sofern sie HBsAg umfasst, in C57B16-Mäusen bei einer Immunisierung gemäß dem hierin beschriebenen Immunisierungsverfahren potente Antikörperantworten gegen HBsAg induziert. Die Gesamt-anti-HBs-Antikörpertiter, ermittelt mittels des hierin beschriebenen AUSAB^{®}-Assays belaufen sich dabei im Mittel auf mindestens 100 mIU/mL, vorzugsweise mindestens 500 mIU/mL, besonders bevorzugt auf mindestens 1.000 mIU/mL und am meisten bevorzugt auf mindestens 1.500 mIU/mL. Die Bestimmung der anti-HBsspezifischen IgG-Isotyp-Klassen zeigt, dass sowohl IgGl- als auch IgG2b-Titer erhöht werden, was als Zeichen einer ausgewogenen Th1/Th2-Immunantwort gilt.

Einen weiteren Beitrag zur Lösung der Eingangs genannten Aufgaben leistet ein Verfahren zur Herstellung einer Zusammensetzung, umfassend die Verfahrensschritte I), II) und IV) und gegebenenfalls III):
I) Bereitstellen eines HBcAg₁₋ₓ's, wobei x eine ganze Zahl aus dem Bereich von 100 bis 160, vorzugsweise dem Bereich von 120 bis 150, besonders bevorzugt dem Bereich von 140 bis 149, darüber hinaus bevorzugt dem Bereich von 144 bis 146 und am meisten bevorzugt gleich 145 ist, eines Fragmentes aus diesem Antigen, einer Variante dieses Antigens oder des Fragmentes dieses Antigens oder einer Mischung aus mindestens zwei davon,
II) Bereitstellen eines Adjuvans beinhaltend ein Saponin, ein Saponinderivat oder eine Mischung aus mindestens zwei davon,
III) Bereitstellen eines HBsAg's, eines Fragmentes aus diesem Antigen, einer Variante dieses Antigens oder des Fragmentes dieses Antigens oder einer Mischung aus mindestens zwei davon, und
IV) In Kontakt bringen des HBcAg₁₋ₓ's, des Adjuvans und gegebenenfalls des HBsAg's.

Als HBcAg₁₋ₓ bzw. als Fragment oder Variante davon, als Adjuvans beinhaltend ein Saponin, ein Saponinderivat oder eine Mischung aus mindestens zwei davon sowie als HBsAg bzw. als Fragmentes oder Variante davon sind diejenigen Verbindungen bevorzugt, die bereits im Zusammenhang mit der erfindungsgemäßen Zusammensetzung als bevorzugte Komponenten i), ii) und iii) genannt wurden.

Hinsichtlich der Verfahrensschritte I) und III) ist es bevorzugt, dass das Bereitstellen des HBcAg's und des HBsAg's dergestalt erfolgt, dass die aus dem Plasma von Patienten mit chronischer Hepatitis B (nur im Falle des HBsAg's), durch synthetische Verfahren oder durch rekombinante Expression gewonnenen Antigene oder Antigenfragmente in einem geeigneten Arzneistoffträger vorgelegt werden. Bei diesem Aizneistoffträger handelt es sich vorzugsweise um eine Flüssigkeit, besonders bevorzugt um eine wässrige Flüssigkeit, darüber hinaus bevorzugt um eine wässrige Salzlösung wie PBS. Vor dem in Kontakt bringen mit den übrigen Komponenten im Verfahrensschritt IV) können die auf diese Weise erhaltenen wässrigen Antigenlösungen durch dem Fachmann bekannte Verfahren sterilfiltiert werden.

Dabei ist es gemäß einer bevorzugten Ausführungsform des erfindungsgemäßen Verfahrens bevorzugt, dass das Bereitstellen des HBcAg's und gegebenenfalls des HBsAg's in den Verfahrensscontten I) und III) dergestalt erfolgt, dass diese Antigene zunächst in einem Arzneistoffträger, vorzugsweise in einer wässrigen Salzlösung, in einer Konzentration vorgelegt werden, die mindestens doppelt, besonders bevorzugt mindestens dreimal, darüber hinaus bevorzugt mindestens viermal und am meisten bevorzugt mindestens sechsmal so groß ist wie die Konzentration des HBcAg's bzw. des HBsAg's in der letztendlich angestrebten erfindungsgemäßen Zusammensetzung (soll also beispielsweise die Konzentration an HBcAg in der erfindungsgemäßen Zusammensetzung 10 µg/ml betragen, so ist es bei der Herstellung der Zusammensetzung bevorzugt, dass das HBcAg zunächst in einer wässrigen Salzlösung in einer Konzentration von mindestens 20 µg/ml, besonders bevorzugt mindestens 30 µg/ml, darüber hinaus bevorzugt mindestens 40 µg/ml und am meisten bevorzugt mindestens 60 µg/ml bereitgestellt wird). Sollen sowohl HBcAg als auch HBsAg in der erfindungsgemäßen Zusammensetzung enthalten sein, so ist es bevorzugt, diese gemeinsam in ein und derselben Salzlösung in den vorstehend genannten Konzentrationen (mindestens 2fach, mindestens 3fach, mindestens 4fach bzw, mindestens 6fach überkonzentriert) vorzulegen. Weiterhin ist es erfindungsgemäß bevorzugt, dass diese überkonzentrierten Antigenlösungen vor dem in Kontakt bringen mit dem Adjuvans im Verfahrensschritt IV) für eine Zeitdauer von 10 Minuten bis 4 Stunden, besonders bevorzugt von 20 Minuten bis 2 Stunden und darüber hinaus bevorzugt von 30 Minuten bis 1 Stunde bei einer Temperatur in einem Bereich von 30 bis 60°C, besonders bevorzugt 35 bis 55°C und darüber hinaus bevorzugt 37 bis 50°C inkubiert werden. Nach dem in Kontakt bringen des Antigens mit dem Adjuvans ist es weiterhin bevorzugt, dass die so erhaltene Zusammensetzung für einen Zeitraum von 15 Minuten bis 5 Stunden, besonders bevorzugt von 30 Minuten bis 3 Stunden und darüber hinaus bevorzugt von 45 Minuten bis 2 Stunden bei einer Temperatur von 10 bis 40°C, besonders bevorzugt von 15 bis 30°C, darüber hinaus bevorzugt von 20 bis 25°C und am meisten bevorzugt bei Raumtemperatur inkubiert wird.

Im Verfahrensschritt II) wird das Adjuvans bereitgestellt. Auch hier ist es bevorzugt, dass das Adjuvans, bevor es mit den Komponenten I) oder III) in Kontakt gebracht wird, zunächst, beispielsweise durch Sterilfiltration, sterilisiert wird, sofern das eingesetzte Adjuvans nicht bereits in steriler Form kommerziell erhältlich ist.

Insbesondere dann, wenn als Adjuvans Komplexe aus Saponinen oder Saponinderivaten, Steroiden und/oder Phospholipiden eingesetzt werden (ISCO-Matrix), ist es erfindungsgemäß bevorzugt, dass zunächst die Komplexe in steriler Form hergestellt bzw. von entsprechenden Anbietern kommerziell erworben werden und anschließend der Saponinkomplex bzw. der Saponinderivatkomplex mit der sterilisierten, wässrigen HBcAg-Lösung und gegebenenfalls mit der sterilisierten HBsAg-Lösung oder aber mit einer Mischung aus diesen beiden Lösungen in Kontakt gebracht wird. Auf diese Weise kommt es nicht zum Aufbau eines klassischen ISCOM-Komplexes, bei dem die Antigene in das Innere der ISCO-Matrix eingebaut werden (sogenannte ISCOMs).

Die so erhaltenen Zusammensetzungen können anschließend noch mit weiteren Zusatzstoffen, wie etwa weiteren Arzneistoffträgern und Hilfsstoffen vermischt werden, je nach dem, ob die Zusammensetzung oral, intranasal, intradermal, subkutan, intramuskulär oder intravenös verabreicht werden soll. Als Arzneistoffträger und Hilfsstoffe sind wiederum diejenigen Verbindungen bevorzugt, die bereits im Zusammenhang mit der erfindungsgemäßen Zusammensetzung als bevorzugte Arzneistoffträger und bevorzugte Hilfsstoffe genannt wurden.

Sofern wässrige Lösungen erhalten werden, können diese in wässrigem, sterilem Zustand verpackt oder lyophylisiert werden, wobei die lyophylisierte Zusammensetzung vor der Verabreichung mit einer sterilen Lösung kombiniert wird.

Einen weiteren Beitrag zur Lösung der Eingangs genannten Aufgaben liefern die durch das vorstehend beschriebene Verfahren erhältlichen Zusammensetzungen, wobei diese Zusammensetzungen vorzugsweise die gleichen Eigenschaften aufweisen wie die eingangs beschriebene, erfindungsgemäße Zusammensetzung.

Einen Beitrag zur Lösung der Eingangs genannten Aufgaben leistet auch eine Arzneimittelformulierung, umfassend die erfindungsgemäße Zusammensetzung sowie mindestens einen der eingangs genannten Zusatzstoffe iv).

Einen Beitrag zur Lösung der Eingangs genannten Aufgaben leistet auch die Verwendung der erfindungsgemäßen Zusammensetzung oder der erfindungsgemäßen Arzneimittelformulierung zur Therapie und/oder zur Prophylaxe von HBV-Infektionen und HBV-vermittelten Erkrankungen. Vorzugsweise wird die erfindungsgemäße Zusammensetzung oder die erfindungsgemäße Armeimittelformuliemng zur Verabreichung an Säugetiere, insbesondere Menschen, eingesetzt. Gemäß einer bevorzugten Verwendung der erfindungsgemäBen Zusammensetzung oder der erfindungsgemäßen Arzneimittelformulierung dient diese insbesondere zur Therapie und/oder zur Prophylaxe, vorzugsweise der Therapie, akuter und/oder chronischer, vorzugsweise chronischer HBV-Infektionen.

Der Begriff "Prophylaxe" im Zusammenhang mit einer HBV-Infektion beim Menschen umfasst dabei vorzugsweise
- die Behandlung eines Menschen, welcher noch keinen Kontakt mit HBV hatte, mit dem Ziel, zu verhindern, dass eine Infektion mit HBV erfolgt-,
- die Behandlung eines Menschen, welcher bereits Kontakt mit HBV hatte und der sich in der immunotoleranten chronischen Phase befindet, mit dem Ziel, zu verhindern, dass dieser Mensch in die immunoaktive chronische Phase eintritt, sowie
- die Behandlung eines Menschen, welcher bereits Kontakt mit HBV hatte und der sich im inaktiven Trägerstadium befindet, mit dem Ziel, zu verhindern, dass dieser Mensch erneut in die immunoaktive Phase eintritt.

Der Begriff "Therapie" im Zusammenhang mit einer HBV-Infektion beim Menschen umfasst dabei vorzugsweise
- die Behandlung eines Menschen, welcher bereits Kontakt mit HBV hatte und der sich in der akuten Phase der Infektion befindet,
- die Behandlung eines Menschen, welcher bereits Kontakt mit HBV hatte und der sich in der immunoaktiven chronischen Phase befindet.

Besonders bevorzugt wird die erfindungsgemäße Zusammensetzung oder die erfindungsgemäße Arzneimittelformulierung Patienten verabreicht, die bereits mit HBV infiziert sind.

Personen in der akuten Infektionsphase, der immunotoleranten chronischen Phase, der immunoaktiven chronischen Phase oder im inaktiven Trägerstadium können mit der erfindungsgemäBen Zusammensetzung oder der erfindungsgemäßen Arzneimittelformulierung je nach Bedarf separat oder in Kombination mit anderen Therapien behandelt werden. Bei therapeutischen Anwendungen wird die Zusammensetzung bzw. die Arzneimittelformulierung in einer Menge an einen Patienten verabreicht, die ausreicht, um eine wirksame CTL-Antwort gegen HBV-Antigene hervorzurufen und HBV-assoziierte Symptome und/oder Komplikationen heilt oder zumindest teilweise stoppt. Wird die erfindungsgemäße Zusammensetzung bzw. die erfindungsgemäße Arzneimittelformulierung, wie besonders bevorzugt, zur Therapie der chronischen Hepatitis B eingesetzt, so ist das Ziel der Therapie eine Reduzierung oder im Idealfall eine Eliminierung virusinfizierter Zellen. Da Personen aufgrund einer inadäquaten oder fehlenden CTL-Antwort während der akuten Phase ihrer Infektion eine chronische Hepatitis B entwickeln können, ist es wichtig, eine Menge der immunrelevanten viralen Antigene (HBcAg₁₋ₓ und gegebenenfalls HBsAg) in einer erfindungsgemäßen Zusammensetzung bzw. der erfindungsgemäßen Arzneimittelformeerung bereitzustellen, die ausreicht, um eine CTL-Antwort wirksam zu stimulieren. Mengen, mit denen diese Ziele erreicht werden, werden als "therapeutisch wirksame Dosen" definiert. Die für den jeweiligen Anwendungsfall therapeutisch wirksame Dosis hängt beispielsweise von der genauen Zusammensetzung der erfindungsgemäßen Zusammensetzung bzw. der erfindungsgemäßen Arzneimittelformulierung, der Verabreichungsart, dem Stadium und der Schwere der behandelten Krankheit, dem Gewicht und dem allgemeinen Gesundheitszustand des Patienten sowie dem Urteil des verschreibenden Arztes ab, liegt im Allgemeinen jedoch vorzugsweise in einem Bereich von etwa 0,1 bis 2.000 µg (Gesamtmenge aus Adjuvans, HBcAg₁₋ₓ und gegebenenfalls HBsAg) für einen 70 kg schweren Patienten, besonders bevorzugt in einem Bereich von 0,5 bis 1.500 µg, darüber hinaus bevorzugt in einem Bereich von 1 bis 1.000 µg und am meisten bevorzugt in einem Bereich von 10 bis 500 µg, gefolgt von Auffrischungsdosen in einem Bereich von 0,1 bis 2.000 µg über Wochen bis Monate, je nach der CTL-Antwort eines Patienten, die durch Messung der HBV-spezifischen CTL-Aktivität in den peripheren Blutlymphozyten (PBL) des Patienten bestimmt wird.

Bei der Therapie der chronischen Hepatitis B sollte die Verabreichung solange fortgesetzt werden, bis zumindest die klinischen Symptome oder die Laborindikatoren anzeigen, dass die HBV-Infektion eliminiert wurde oder zumindest unter der Kontrolle des Immunsystems ist, gegebenenfalls noch länger.

Die erfindungsgemäße Zusammensetzung bzw. die erfindungsgemäße Arzneimittelformulierung kann grundsätzlich oral, intranasal, intradermal, subkutan, intramuskulär oder intravenös verabreicht werden.

Einen Beitrag zur Lösung der Eingangs genannten Aufgaben leistet weiterhin die Verwendung der erfindungsgeznäDen Zusammensetzung zur Herstellung einer Aizneimittelformulierung zur Therapie und/oder zur Prophylaxe von HBV-Infektionen und HBV-vermittelten Erkrankungen.

Des weiteren leistet ein Verfahren zur Therapie und/oder zur Prophylaxe von HSV-Infektionen und HBV-vermittelten Erkrankungen einen Beitrag zur Lösung der Eingangs genannten Aufgaben, bei dem einem noch nicht mit HBV in Kontakt getretenen Menschen oder einem bereits mit HBV in Kontakt getretenen Menschen, vorzugsweise einem an chronischer Hepatitis B leidenden Patienten, eine therapeutisch wirksame Dosis der erfindungsgemäßen Zusammensetzung bzw. der erfindungsgemäßen Arzneimittelformulierung vorzugsweise in der vorstehend beschriebenen Art und Weise verabreicht wird.

Die Erfindung wird nun anhand nicht limitierender Figuren und Beispiele näher erläutert.

Die Figur 1 zeigt die CTL-Antworten bei der Verwendung erfindungsgemäßer Zusammensetzungen mit AbISCO®-100 als Adjuvans.

Die Figur 2 zeigt die CTL-Antworten bei der Verwendung erfindungsgemäßer Zusammensetzungen mit AbISCO®-200 als Adjuvans.

Figuren 3A und 3B zeigen die B-Zell-Antworten bei der Verwendung erfindungsgemäßer Zusammensetzungen, welche HBcAg und HBsAg beinhalten, mit AbISCO®-200 als Adjuvans.

### METHODEN

### Immunisierung von C57B1/6-Mäusen

C57B1/6-Mäusen wurden mit den in den Tabellen 1 und 2 angegebenen Mengen an PBS, HBcAg, HBsAg und Adjuvans in jeweils 100 µl PBS immunisiert, wobei jeweils zweimal im Abstand von 21 Tagen die jeweils angegebenen Mengen subkutan injiziert wurden. Für die DNA Immunisierung wurde das Plasmid pCl/HBV_{ayw}core in Mengen von 2 ×50 µg in jeweils 50 µL PBS in je einen *Tibialis anterior*-Muskel injiziert.

### Bestimmung der antigen-spezifischen CD8⁺-T-Zell-Freauenzen in der Milz

Zwei Wochen nach der zweiten Immunisierung wurden den Tieren die Milzen entnommen und Zellsupensionen aus den Milzzellen hergestellt. Die Herstellung von Milzzellsuspemionen ist in Schirmbeck R., Böhm W., Fissolo N., Melber K., und Reimann J.: "Different imunogenicity of H-2 Kb-restriucted epitopes in natural variants of the hepatitis B surface antigen", Europ. J. Immunonol. 2003, 33: 2429-38 beschrieben. Die Milzzellen wurden für 4 Stunden in RPMI-1640 Medium mit 0,25 µM HBcAg-spezifischem Peptid (MGLKFRQL; Jerini BioTools, Berlin, DE) und 5 µg/ml Brefeldin A (BFA) (Katalognr. 15870, Sigma) bei 37°C inkubiert (Restimulation). Die restimulierten Zellen wurden geerntet und deren Oberfläche mit anti-CD8 mAb (anti-Maus CD8a-PE-Konjugat; Katalognr. 55303; BD Biosciences, Heidelberg, DE) gefärbt, fixiert, permeabilisiert und eine Färbung auf cytoplasmatisches IFN_{γ} (anti-Maus IFN_{γ}-FITC-Konjugat; Katalognr. 554411; BD-Biosciences, Heidelberg, DE) durchgeführt. Die Frequenzen von core- spezifischen CD8⁺IFN_{γ}⁺CTL's wurden durch FACS Analyse bestimmt. Der Durchschnittswert für CD8⁺IFN_{γ}⁺-T-ZeUen/10⁵ CD8⁺T-Zellen ist gezeigt.

### Bestimmung des Antikörper- Isotyps

96-well-Mikrotiterplatten (Nunc, Wiesbaden, DE) wurden mit dem zur Immunisierung eingesetzten HBsAg (2 µg/mL in Carbonatpuffer) über Nacht bei 4°C beschichtet (100 µL pro Vertiefung).

Zu Beginn der ELISA-Durcbfuhrtmg wurden die beschichteten Platten dreimal mit PBST (Phosphat-gepufferte Salzlösung mit 0.5 % Tween 20) gewaschen. Die Vertiefungen wurden mit 0.2 % Gelatine 1 h bei 37°C geblockt. Die zu bestimmenden Mausplasmen/ -seren wurden in 2er-Stufen in PBS verdünnt (im Bereich 1:250 bis 1:32 000) und jeweils 100 µL der Verdünnungen in die entsprechenden Vertiefungen gegeben (in Duplikaten) und 1 h bei 37°C inkubiert. Als Kontrolle diente Serum von nicht immunisierten Mäusen. Anschließend wurde die Mikrotiterplatte fünf Mal mit PBST gewaschen und mit einem Isotyp-spezifischen Zweitantikörper (Peroxidase-konjugiert) inkubiert. Zum Nachweis von IgG1 wurde gamIg1 (Katolognr. GAM/IgG1/PO) und zum Nachweis von IgG2b gamIg2b (Katalognr. IgG2b/PO; Nordic Immunological Laboratories, Tilburg, Niederlande) eingesetzt. Anschließend wurde die Farbreaktion mit o-Phenylenediamin (oPD) (Sigma; Katalognr. P2903) als Substrat (1 mg/ml oPD mit 1 µl/mL 30 % H₂O₂ durchgeführt (100 µL pro Vertiefung). Die Farbreaktion wurde nach 10-15 Minuten mittels 1 N H₂SO₄ (50 µL pro Vertiefung) gestoppt und die optischen Dichte bei 492nm im Spektrophotometer (SpectraMax plus; Molecular Devices, Ismaning, DE) gemessen.

### Bestimmung des Gesamt-anti-HBs- Antikörpertiters

Der anti-HBs-Antikörpertiter in den Seren der immunisierten Mäuse wurde bestimmt im IMX Automated Immunoassay Analyzer (Abbott Diagnostics, Wiesbaden, DE) unter Verwendung des IMX AUSAB^{®} Testkits (Referenznr. 2226-21, Abbott, Wiesbaden, DE). Die Durchführung des Assays erfolgte wie vom Hersteller beschrieben.

### Herstellung der Antigene

a) HBcAg₁₋₁₄₄₊₁
   Über *Bam*HI-Restriktion wurde aus dem Vektor pBR322 (Bolivar F., Rodriguez R.L., Greene P.J., Betlach M.C., Heyneker H.L., Boyer H.W.: "Construction and Characterization of new cloning vehicles. II. A multipurpose Cloning System", Gene. 1977; 2(2): 95-113) und dem zirkularisierten Hepatitis B Virus Genom (HBV320, 3.182 Basenpaare; Bichko V, Pushko P, Dreilina D, Pumpens P, Gren E (1985), "Subtype ayw variant of hepatitis B virus", FEBS 185: 208-212) das Plasmid pHB320 (7.543 Basenpaare) konstruiert, welches das komplette HBV Genom trägt.
   Nach *Hha*I-Restriktion und S1-Nuklease-Abbau der überhängenden Einzelstrangsequenzen wurde ein 1.000 Basenpaare langes Fragment, das die Gensequenzen für das pre-core/core-Cren (*core open reading frame*) trägt, in den Vektor pBR322 P*trp* (Ikehara M, Ohtsuka E, Tokunaga T et al. (1984) Synthesis of a gene for human growth hormone and its expression in E. coli. Proc. Natl. Acad. Sci. USA 81:5956-5960) über Blunt-end-Ligation kloniert. Dieser wurde zuvor *Bam*HI/*Sal*I geschnitten und die überstehenden Enden mit DNA-Polymerase aufgefüllt. Das resultierende Plasmid pGC74 (6.046 Basenpaare) trägt den Tryptophan-Promotor und das HBc-Gen in entgegengesetzter Orientierung. Diese Orientierung ermöglicht die nachfolgenden Klonierungsschritte durch die Rekonstitution singulärer Schnittstellen zur Optimierung der HBcAg-Expressiorrskassette.
   Zum Wechsel der Orientierung des HBc-Fragmentes wurde pGC74 mit *Bam*HI restringiert, die überstehenden Enden mit DNA-Polymerase aufgefüllt und mit *Sal*I nachgeschnitten. Dieses Fragment wurde mit dem Vektor pBR322 Ptrp ligiert. Dieser wurde zuvor mit *Ava*I linearisiert, die Enden mit DNA-Polymerase aufgefüllt und mit *Sal*I nachgeschnitten. Das so konstruierte Plasmid pGC2 (5.552 Basenpaare) trägt den Tryptophan-Promotor und das HBc-Gen in gleicher Orientierung.
   Dieses Plasmid wurde mit SalI geschnitten, die überstehenden Enden mit der Nuklease *Bal*31 entfernt, mit *Eco*RI nachgeschnitten, die dabei entstehenden überhängenden Enden mit DNA-Polymerase aufgefüllt und erneut ligiert. Es resultierte Plasmid pHBc10 (4.825 Basenpaare), mit einer verkürzten Sequenz zwischen Tryptophan-Promotor und HBc-Gen zum Erhalt einer optimalen P*trp*-Shine-Dalgarno-HBc-Struktur bzw. Distanz.
   Zur Optimierung der Genexpression wurde ein 1.044 Basenpaar-*Hha*I-HBc-Fragment aus pHBc10 nach Restriktion mit *Hha*lI mit S1-Nuklease verdaut und in den Vektor pBR322-*trp*-I, ein modifiziertes pBR322-Plasmid, kloniert. Aus dieser Konstruktion resultierte pHBc3 (7.108 Basenpaare), mit der optimierten Expressionssequenz des HBc-Gens unter der Kontrolle des Tryptophan-Prvmotors.
   Das Plasmid pHBc3 wurde mit *Kpn*21 geschnitten und in die Schnittstelle an der Aminosäureposition 144 des HBc-Gens ein synthetischer Polylinker *Eco*RV*-Cla*I*-Pvu*I (5'-TCCGGAGATATCGATCGTCCGGA-3') eingesetzt. Hieraus resultierte Plasmid pHBc1615 (7.129 Basenpaare). pHBc1615 wurde mit *Cla*I linearisiert und ein synthetischer Polylinker mit einem Terminationscodvn nach der Aminosäureposition 144 sowie einem zusätzlichen Isoleucin-Codon an Position 145 eingesetzt Es wurde Plasmid pHBc2-7 erhalten.
   Kompetente E. coli Zellen (Stamm BL21: Genotyp B F⁻dcm ompT hsdS (r_{B}⁻m_{B}⁻)gal) wurden mit Plasmid pHBc2-7 transformiert und nach Plattierung auf LB Agar mit Ampicillin über Nacht bei 37°C erhalten. Mit jeweils einer Einzelkolonie werden 300 ml M9 Minimalmedium (unter Zusatz von 1 % Casaminosäuren, Difco Kat.-Nr. 223050, Becton-Dickinson, Heidelberg) und 0,2 % Glukose beimpft. Die Kultivierung erfolgte über Nacht bei 37°C auf einem Rundschüttler in 1 1-Erlenmeyerkolben. Die Kulturen erreichten üblicherweise eine optische Dichte (OD₅₄₀ₙₘ) zwischen 2 und 5.
   Die Zellen wurden abzentrifugiert und das Pellet in Lysepuffer (50 mM TRIS/HCl pH 8,0; 5 mM EDTA, 100 µg/ml PMSF, 0,08 % Triton-X-100) resuspendiert. Anschließend wurden die konditionierten Zellen auf Eis durch Ultraschallbehandlung (3 × 15 Sekunden bei 22 kHz) lysiert. Nach Abaentrifugieren der Zelldebris wurden die Proteine aus dem Überstand mit Ammoniumsulfat (33 % Sättigung) für 4-20 Stunden bei 4°C präzipitiert. Das Pellet wurde in PBS mit 0,1 % Triton-X-100 resuspendiert und anschließend jeweils 5-10 ml der erhaltenen Lösung auf eine Sepharose CL4B-Chromatographiesäule (2,5×85 cm) aufgeladen. Die Elution erfolgte mit PBS ohne Zusatz von Triton-X-100, Die Anwesenheit von HBcAg-Polypeptiden in den Einaelfraktionen wurde mittels Polyacrylamidgelelektrophorese / Coomassie-Färbung untersucht. Positive Fraktionen wurden vereinigt und mittels Ammoniumsulfat-Präzipitation (33 % Sättigung) für 20 Stunden bei 4°C konzentriert. Die Proteinpellets wurden in PBS mit einer Konzentration zwischen 3-20 mg/ml resuspendiert und anschließend über Nacht dialysiert gegen 1.000 Volumina des gleichen Puffers. Das HBcAg₁₋₁₄₄₊₁ in PBS wurde sterilfiltriert und bei -20°C gelagert.
b) HBsAg
   HBsAg wurde hergestellt wie beschrieben in: Brocke P, Schaefer S, Melber K, Jenzelewski V, Müller F, Dahlems U., Bartelsen O., Park KN, Janowicz ZA, Gellissen G: "Recombinant hepatitis B vaccines: disease characterization and vaccine production" (2005) in Gellissen G (Herausgeber): "Production of recombinant proteins - novel microbial and eucaryontic expression systemes, Seiten 319-360, Wiley-VCH, Weinheim) und kann von Rhein Biotech, Düsseldorf, Germany, bezogen werden.
c) HBcAg₁₋₁₈₃
   HBcAg₁₋₁₈₃ wurde von DiaSorin S.r.l. (Saluggia, Italien) erhalten.

### BEISPIEL 1

In einer ersten Versuchsreihe wurde als Adjuvans der unter der Produktbezeichnung AbISCO®-100 (ISCONOVA, Uppsala, Schweden) kommerziell erhältliche Saponinkomplex als Adjuvans eingesetzt. Als Positiv-Kontrolle diente core-Plasmid-DNA, als Negativkontrolle reines PBS, reines Adjuvans in PBS, reines HBc₁₋₁₄₄₊₁ in PBS sowie reines HBc₁₋₁₈₃ in PBS. Die erfindungsgemäße Zusammensetzung beinhaltete PBS, HBc₁₋₁₄₄₊₁ sowie AbISCO®-100. Die Mengen der einzelnen Komponenten sind in der Tabelle 1 angegeben.

Das Plasmids pCI/HBV_{ayw}core wurde wie in Kuhröber A, Pudollek HP, Reifenberg K, Chisari FV, Schlicht HJ, Reimann J, Schirmbeck R (1996): "DNA Immunization induces antibody and cytotoxic T cell responses to hepatitis B core antigen in H-2b mice", J Immunol 156: 3687-95, beschrieben, hergestellt (Das Konstrukt wird dort als pCMV-1/c bezeichnet). und mit QIAGEN-tip 500 (Katalognr. 12162; Qiagen, Hilden, Deutschland) wie vom Hersteller beschrieben isoliert.

Für die Formulierung der erfindungsgemäßen Zusammensetzung wurde das HBc₁₋₁₄₄₊₁ zunächst mit PBS auf die 4-fache Konzentration des finalen Ansatzes verdünnt und 30 Minuten bei 37°C unter Schütteln inkubiert. Anschließend wurde AbISCO®-100, so wie vom Hersteller als wässrige Dispersion geliefert, zugefügt und mit PBS die in der Tabelle 1 angegebene Endkonzentration des Antigens eingestellt, wobei sich die in der Tabelle angegebenen Konzentrationen des Adjuvans und des Antigens ergaben. Die so hergestellte Zusammensetzung wurde für eine Stunde bei Raumtemperatur (20-25°C) inkubiert, anschließend über Nacht (12-18 Stunden) bei 2-8°C gelagert und am nächsten Tag zur Immunisierung eingesetzt.

**Tabelle 1**

| Mäusegruppe | Zur Immunisierung eingesetzte Zusammensetzung |
|---|---|
| A (n. erf.¹⁾) | 50 µg core-Plasmid-DNA in 50 µl PBS (Positivkontrolle) |
| B (n. erf.) | 100 µl PBS (Negativkontrolle) |
| C (n. erf.) | 12 µg AbISCO^{®}-100 in 100 µl PBS (Negativkontrolle) |
| D (n. erf.) | 10 µg HBcAg₁₋₁₄₄₊₁ in 100 µl PBS (Negativkontrolle) |
| E (erf.²⁾) | 12 µg AbISCO^{®}-100 und 10 µg HBcAg₁₋₁₄₄₊₁ in 100 µl PBS |

| | |
|---|---|
| ¹⁾ n. erf. = nicht erfindungsgemäß ²⁾ erf. = erfindungsgemäß | |

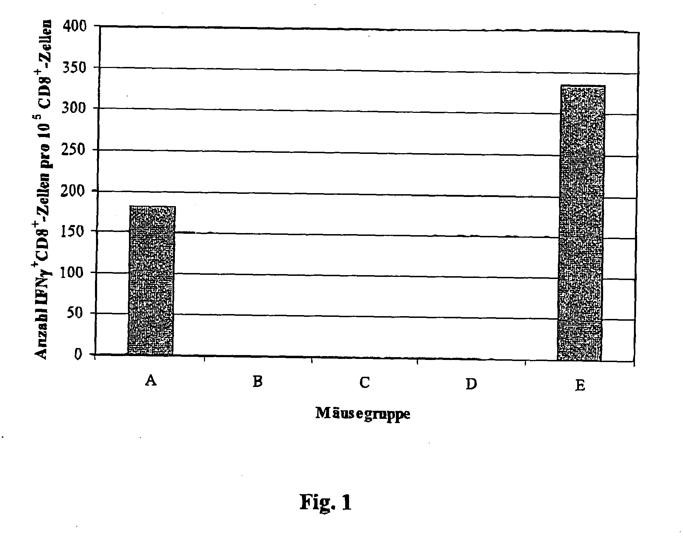

Die durch die vorstehend beschriebenen Zusammensetzungen erzielten CTL-Antworten sind der Figur 1 zu entnehmen. Aus dieser Figur wird ersichtlich, das sich mittels der erfindungsgemäßen Zusammensetzung (Mäusegruppe E) core-spezifische CTL-Antworten erzielen lassen, die sogar stärker sind als diejenigen CTL-Antworten, die mit core-Plasmid-DNA erzielt werden können (Mäusegruppe A).

### BEISPIEL 2

In einer weiteren Versuchsreihe wurde als Adjuvans der unter der Produktbezeichnung AbISCO®-200 (ISGONOVA, Uppsala, Schweden) kommerziell erhältliche Saponinkomplex als Adjuvans eingesetzt. Als Positiv-Kontrolle diente wiederum core-Plasmid-DNA, als Negativkontrolle reines PBS, reines Adjuvans in PBS, reines HBc₁₋₁₄₄₊₁ in PBS sowie reines HBc₁₋₁₈₃ in PBS. Die erfindungsgemäße Zusammensetzung beinhaltete PBS, HBc₁₋₁₄₄₊₁ sowie AbISCO®-200, und gegebenenfalls HBsAg. Die Mengen der einzelnen Komponenten sind in der Tabelle 2 angegeben.

Für die Formulierung der erfindungsgemäßen Zusammensetzung wurde das HBc₁₋₁₄₄₊₁, und gegebenenfalls das HbsAg zunächst mit PBS auf die 4-fache Konzentration des finalen Ansatzes verdünnt und 30 Minuten bei 37°C unter Schütteln inkubiert. Bei der Verwendung von HBsAg wurden zunächst unter aseptischen Bedingungen HBsAg und HBcAg_{1-144+J} zusammengegeben und ebenfalls mit PBS auf die 4-fache Konzentration des finalen Ansatzes verdünnt. Anschließend wurde AbISCO®-200, so wie vom Hersteller als wässrige Dispersion geliefert, zugefügt und mit PBS die in der Tabelle 2 angegebene Endkonzentration des Antigens eingestellt, wobei sich die in der Tabelle angegebenen Konzentrationen des Adjuvans und des Antigens bzw. der Antigene ergaben. Die so hergestellte Zusammensetzung wurde für eine Stunde bei Raumtemperatur (20-25°C) inkubiert, anschließend über Nacht (12-18 Stunden) bei 2-8°C gelagert und am nächsten Tag zur Immunisierung eingesetzt.

**Tabelle 2**

| Mäusegruppe | Zur Immunisierung eingesetzte Zusammensetzung |
|---|---|
| F (n. erf.) | 50 µg core-Plasmid-DNA in 50 µl PBS (Positivkontrolle) |
| G (n. erf.) | 100 µl PBS (Negativkontrolle) |
| H (n. erf.) | 5 µg AbISCO®-200 in 100 µl PBS (Negativkontrolle) |
| I (n. erf.) | 10 µg HBcAg₁₋₁₄₄₊₁ in 100 µl PBS (Negativkontrolle) |
| J (n. erf.) | 10 µg HBcAg₁₋₁₄₄₊₁ und 5 µg HBsAg in 100 µl PBS (Negativkontrolle) |
| K (n. erf.) | 10 µg HBcAg₁₋₁₈₃ in 100 µl PBS (Negativkontrolle) |
| L (erf.) | 5 µg AbISCO^{®}-200 und 10 µg HBcAg₁₋₁₄₄₊₁ in 100 µl PBS |
| M (erf.) | 5 µg AbISCO^{®}-200, 10 µg HBcAg₁₋₁₄₄₊₁ und 5 µg HBsAg in 100 µl PBS |

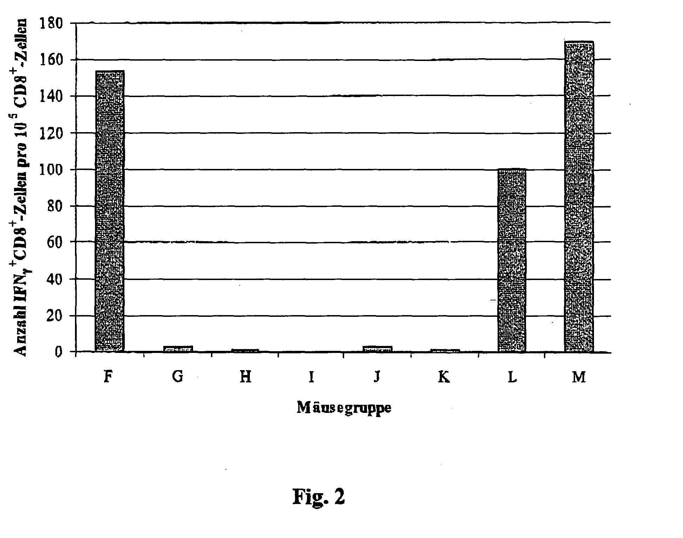

Die durch die vorstehend beschriebenen Zusammensetzungen erzielten CTL-Antworten sind der Figur 2 zu entnehmen. Auch aus dieser Figur wird ersichtlich, das sich mittels der erfindungsgemäßen Zusammensetzung (Mäusegruppe L und M) core-spezifische CTL-Antworten erzielen lassen, die vergleichbar sind mit denjenigen CTL-Antworten, die mit core-Plasmid-DNA erzielt werden können (Mäusegruppe F).

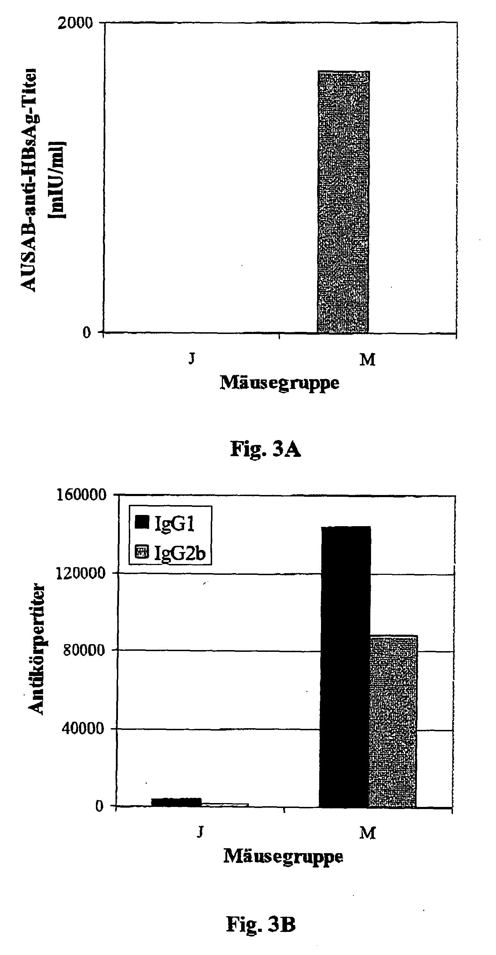

Der Figur 3A ist zu entnehmen, dass die erfindungsgemäße Zusammensetzung, sofern sie auch HBsAg umfasst, auch zu einer potenten Antikörperantwort gegen HBsAg führt. Die Figur 3B wiederum zeigt, dass durch eine solche erfindungsgemäße Zusammensetzung sowohl IgG1- als auch IgG2b-Titer erhöht werden, was als Zeichen einer ausgewogenen Th1/Th2-Immunantwort gilt.

### Annex zu den Anmeldungsunterlagen - nachgereichtes Sequenzprotokoll

## Patentansprüche

1. Eine Zusammensetzung, beinhaltend:
i) ein HBcAg₁₋ₓ, wobei x eine ganze Zahl aus dem Bereich von 100 bis 160 ist, ein Fragment aus diesem Antigen, eine Variante dieses Antigens oder des Fragmentes dieses Antigens oder mindestens zwei davon
ii) ein Adjuvans beinhaltend ein Saponin oder ein Saponinderivat oder eine Mischung aus mindestens zwei davon,
sowie gegebenenfalls
iii) ein HBsAg, ein Fragment aus diesem Antigen, eine Variante dieses Antigens oder des Fragmentes dieses Antigens oder mindestens zwei davon.

2. Die Zusammensetzung nach Anspruch 1, wobei x eine ganze Zahl aus dem Bereich von 140 bis 149 ist.

3. Die Zusammensetzung nach Anspruch 1 oder 2, wobei x eine ganze Zahl aus dem Bereich von 144 bis 146 ist.

4. Die Zusammensetzung nach einem der vorhergehenden Ansprüche, wobei das Adjuvans ein Saponinkomplex, ein Saponinderivatkomplex oder eine Mischung von mindestens zwei davon ist und als von Wasser oder wässrigen Salzlösungen verschiedene Komponenten
(α1) 0 bis 95 Gew.-% eines Phospholipids,
(α2) 0 bis 95 Gew.-% eines Steroids,
(α3) 5 bis 100 Gew.-% des Saponins oder Saponinderivates, sowie
(α4) 0 bis 20 Gew.-% weitere Zusatzstoffe
beinhaltet, wobei die Gewichtsmengen jeweils auf das Gesamtgewicht des Saponinkomplexes bzw. des Saponindetivatkomplexes, mit Ausnahme seines Wasseranteils bzw. seines Anteils an wässrigen Salzlösungen, bezogen sind und die Summe der Komponenten (α1) bis (α4) 100 Gew.-% beträgt.

5. Die Zusammensetzung nach einem der vorhergehenden Ansprüche, wobei das Adjuvans eine ISCO-Matrix ist.

6. Die Zusammensetzung nach einem der vorhergehenden Ansprüche, wobei das relative Mengenverhältnis zwischen der Antigenkomponente i) bzw. den Antigenkomponenten i) und iii) zum Adjuvans ii) in einem Bereich von 1 : 20 bis 20 : 1 liegt.

7. Die Zusammensetzung nach einem der vorhergehenden Ansprüche, wobei die Gesamtkonzentration der Komponenten i), ii) und gegebenenfalls iii) in der Zusammensetzung in einem Bereich von 0,1 bis 2.000 µg/ml liegt.

8. Die Zusammensetzung nach Anspruch 1, umfassend
i) ein HBcAg₁₋₁₄₅ sowie
ii) einen Saponinkomplex.

9. Die Zusammensetzung nach Anspruch 1, umfassend
i) ein HBcAg₁₋₁₄₄₊₁ sowie
ii) einen Saponinkomplex.

10. Die Zusammensetzung nach Anspruch 1, umfassend
i) ein HBcAg₁₋₁₄₅,
ii) einen Saponinkomplex, sowie
iii) ein HBsAg.

11. Die Zusammensetzung nach Anspruch 1, umfassend
i) ein HBcAg_{1-144+1,}
ii) einen Saponinkomplex, sowie
iii) ein HBsAg.

12. Ein Verfahren zur Herstellung einer Zusammensetzung, umfassend die Verfahrensschritte I), II) und IV) und gegebenenfalls III):
I) Bereitstellen eines HBcAg₁₋ₓ's, wobei x eine ganze Zahl aus dem Bereich von 100 bis 160 ist, eines Fragmentes aus diesem Antigen, einer Variante dieses Antigens oder des Fragmentes dieses Antigens oder einer Mischung aus mindestens zwei davon,
II) Bereitstellen eines Adjuvans beinhaltend ein Saponin, ein Saponinderivat oder eine Mischung aus mindestens zwei davon,
III) Bereitstellen eines HBsAg's, eines Fragmentes aus diesem Antigen, einer Variante dieses Antigens oder des Fragmentes dieses Antigens oder einer Mischung aus mindestens zwei davon, und
IV) In Kontakt bringen des HBcAg₁₋ₓ's, des Adjuvans und gegebenenfalls des HBsAg's.

13. Das Verfahren nach Anspruch 12, wobei x eine ganze Zahl aus dem Bereich von 140 bis 149 ist.

14. Das Verfahren nach Anspruch 12 oder 13, wobei x eine ganze Zahl aus dem Bereich von 144 bis 146 ist.

15. Das Verfahren nach einem der Ansprüche 12 bis 14, wobei das Adjuvans ein Saponinkomplex, ein Saponinderivatkomplex oder eine Mischung von mindestens zwei davon ist und als von Wasser oder wässrigen Salzlösungen verschiedene Komponenten
(α1) 0 bis 95 Gew.-% eines Phospholipids,
(α2) 0 bis 95 Gew.-% eines Steroids,
(α3) 20 bis 100 Gew.-% des Saponins oder Saponinderivates, sowie
(α4) 0 bis 20 Gew.-% weitere Zusatzstoffe
beinhaltet, wobei die Gewichtsmengen jeweils auf das Gesamtgewicht des Saponinkomplexes bzw. des Saponinderivatkomplexes, mit Ausnahme seines Wasseranteils bzw. seines Anteils an wässrigen Salzlösungen, bezogen sind und die Summe der Komponenten (α1) bis (α4) 100 Gew.-% beträgt.

16. Das Verfahren nach einem der Ansprüche 12 bis 15, wobei das Adjuvans eine TSCO-Matrix ist

17. Eine Zusammensetzung, erhältlich durch ein Verfahren nach einem der Ansprüche 12 bis 16.

18. Eine Arzneimittelformulierung, umfassend die Zusammensetzung nach einem der Ansprüche 1 bis 11 oder 17 sowie geeignete Zusatzstoffe.

19. Die Verwendung der Zusammensetzung nach einem der Ansprüche 1 bis 11 oder 17 zur Herstellung einer Arzneimittelformulierung zur Therapie und/oder zur Prophylaxe von HBV-Infektionen und HBV-vermittelten Erkrankungen.

20. Die Verwendung der Zusammensetzung nach einem der Ansprüche 1 bis 11 oder 17 zur Herstellung einer Arzneimittelformulierung zur Behandlung chronischer HBV-Infektionen.


SEQUENCE LISTING

<110>  Rhein Biotech GmbH

<120>  Eine Zusammensetzung zur Therapie und/oder zur Prophylaxe von
HBV-Infektionen und HBV-vermittelten Erkrankungen

<130>  RB82336EP

<140>  05020208.4
<141>  2005-09-16

<160>  2

<170>  PatentIn version 3.1

<210>  1
<211>  8
<212>  PRT
<213>  synthetic polypeptide

<400>  1

Met Gly Leu Lys Phe Arg Gln Leu
1               5

<210>  2
<211>  23
<212>  DNA
<213>  synthetic polynucleotide

<400>  2
tccggagata tcgatcgtcc gga                                              23